# EUROPEAN PATENT APPLICATION

(11) **EP 4 792 766 A1**
(43) Date of publication of application: **19.08.2026**
(21) Application number: 26155334.1
(22) Date of filing: 30.01.2026
(51) Int. Cl.: A61B 34/10

(54) **METHOD FOR PLANNING KNEE REPLACEMENT SURGERY COMPRISING PROXIMAL AND LOCAL MORPHOLOGICAL, PROSTHETIC DESIGN AND PROSTHEITC POSITION RELATED PARAMETERS**

(30) Priority: 12.02.2025 BE 202505082; 02.04.2025 BE 202505205
(71) Applicant: Arthro ID, 8890 Moorslede (BE)
(72) Inventor: Luyckx, Thomas, 8890 Moorslede (BE); Meyer, Andrew, 8890 Moorslede (BE); Grammens, Jonas, 8890 Moorslede (BE); Verstraete, Matthias, 8890 Moorslede (BE)
(74) Representative: Willnegger, Eva

(57) **Abstract**

A computer-implemented method of planning knee replacement surgery, the method comprising:
▪ Receiving input related to an anatomy of a patient, wherein the input comprises imaging data related to the femur, the tibia, the patella and optionally the hip, pelvis, the fibula, the ankle, and/or the bones in the foot;
▪ Generating a three-dimensional (3D) model of the anatomy based on the input, wherein the 3D model comprises a patella model, a model of the femur, the tibia and optionally of the fibula, the pelvis, the hip, the ankle and the bones in the foot;
▪ Characterizing a morphology of the patella, the femur, the tibia, and optionally the fibula, the hip, the pelvis, the ankle and the bones in the foot based on the 3D model;
▪ Determining one or more implant-related or morphological parameters based on one or more of the 3D model and the morphology;
▪ Performing one or more morphological and/or biomechanical simulations based on the one or more implant-related or morphological parameters, wherein the simulations include one or more poses of the patella-femoral and tibio-femoral joint through a range of motion thereof and optionally a range of motion of the hip and/or the ankle;
▪ Optimizing a position, an orientation and optionally a size of the femur implant relative to the native femur and patella based on the one or more morphological and/or biomechanical simulations;
▪ Optionally optimizing a position, an orientation and optionally a size of the tibia implant relative to the femur implant, femur implant position and patella based on the one or more morphological and / or biomechanical simulations; and
▪ Outputting patient-specific planning information to one or more of an external computing device, visual display and a computer-readable storage device.

## Description

### FIELD OF THE INVENTION

Total knee arthroplasty (TKA) procedures aim to replace the knee joint using implants and to restore the function of the natural knee joint. Techniques and tools for correctly positioning and orienting femoral and tibial components in TKA are constantly being improved. Examples include patient-specific instruments (PSI), femoral and tibial custom-made cutting guides, femoral and tibial pre-operative templating tools, and surgical navigation systems.

### BACKGROUND

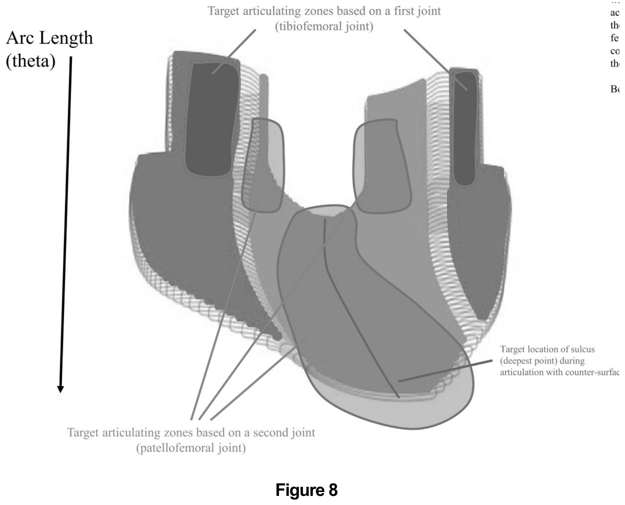

Figure 8 of WO2022076773A1 to Smith & Nephew teaches a planning method for patellar replacements based on 3D model. Biomechanical measurements of the patellofemoral joint are determined including a mechanical axis and preoperative leg deformity. A 3D model of the patient anatomy is generated based on the input, and the 3D model is characterized in terms of the morphology of the patella. An implant is sized and fitted to the 3D model and implant position and orientation are optimized based on the biomechanics. Results are outputted as a patient report or a surgical plan to a computing device and/or a storage medium. But this document does not consider the relative position of the femur axis, the femur implant axis and the patella axis as well as the articulating surfaces in view of ensuring a sufficient gap between the femur implant and the patella. This document does not account for landmarks at the acetabulum or the torsion of the femur in the planning of the patellar implant / femoral implant position nor does it take into account the trochlear anatomy.

WO2021149000A2 to Symbios Orthopédie is directed at a method for making of a personalized implant considering the preoperative condition, the prearthritic condition, and the target postoperative HKA alignment to define an individually adapted knee prothesis. But this document does not disclose considering the relative position of the femur axis, the femur implant axis and the patella axis as well as the articulating surfaces in view of ensuring a sufficient gap or distance between the femur implant and the patella.

WO2023118200A1 pending to Ostesys discloses a planning tool for a knee implant placement based on a 3D model of the tibia and the femur and alignment parameters, such as the hip-knee-ankle angle (HKA), the lateral patellar tile (LPT) and the lateral patellar shift (LPS). But this document does not disclose considering the relative position of the femur axis, the femur implant axis and the patella axis as well as the articulating surfaces in view of ensuring a sufficient gap between the femur implant and the patella.

More generally, it is acknowledged that the patella can be treated in a variety of ways during knee joint replacement surgery. The surgeon may decide to remove the defective cartilage and replace it with a poly-ethylene artificial component to improve the patellar function. Rarely, however, is the relationship with the femoral component considered and the impact of the femoral component orientation and position on the patellar functioning.

However, despite these efforts, many patients still suffer from short-term and long-term post-operative pain after partial or total knee replacement surgery.

Therefore, there is still a need for an improved method of automating and/or supporting the knee replacement workflow to streamline the preoperative and the intraoperative decision making, improve surgical outcomes, and reduce the risk of patellofemoral complications. More specifically, there is a need for improved metrics and measurements that quantify the placement of implants required to better restore healthy function of the knee taking into account the complexity of the knee and its surrounding joints. This invention details the system and novel method of planning a TKA surgery accounting for these novel anatomical measurements.

### SHORT DESCRIPTION OF THE INVENTION

The present inventors surprisingly found that the short-term and the long-term post-operative pain in TKA can be reduced by correcting the gap and/or position between the femur implant and the patella, preferably between the femur and the femur implant and the patella and optionally the patella implant.

Accordingly, the present inventors propose a planning tool for total or partial knee replacement arthroplasty. The planning tool considers a variety of parameters.

Importantly, the planning tool considers the relative position of the patella axis, the femur axis and the femur implant axis as well as the articulating surfaces between the femur implant and the patella, preferably the articulating surfaces between the fmur, the femur implant and the patella and optionally the patella implant. The planning tool further considers the hip and pelvis position, and preferably the orientation and optionally the patella implant position and orientation, so to enable the correction of the femur implant position and orientation in view of ensuring a sufficient gap between the femur implant and the patella, or the patella implant.

Accordingly, a first aspect of the invention is a computer-implemented method of planning knee replacement surgery, the method comprising:
▪ Receiving input related to an anatomy of a patient, wherein the input comprises imaging data related to the femur, preferably the proximal femur, optionally the distal femur, the tibia, preferably the proximal tibia, preferably the distal tibia, the patella, optionally the proximal fibula, the distal fibula, the pelvis and optionally the hip, pelvis, the fibula, the ankle, and/or the bones in the foot;
▪ Generating a three-dimensional (3D) model of the anatomy based on the input, wherein the 3D model comprises a patella model, a model of the femur, the tibia and optionally of the fibula, the pelvis, the hip, the ankle and the bones in the foot;
▪ Characterizing a morphology of the patella, the femur, the tibia, and optionally the fibula, the hip, the pelvis, the ankle such as the calcaneum and the talus and the bones in the foot based on the 3D model;
▪ Optionally receiving a library of implant models with varying size and shape whereby each of the implant models is represented by a 3D model;
▪ Determining one or more implant-related or morphological parameters, preferably one or more implant-related and morphological parameters, based on one or more of the 3D model and the morphology;
▪ Performing one or more morphological and/or biomechanical simulations, preferably one or more morphological and biomechanical simulations, based on the one or more implant-related or morphological parameters, wherein the simulations include one or more poses of the patella-femoral and tibio-femoral joint, preferably of the knee joint, through a range of motion thereof and optionally a range of motion of the hip and/or the ankle;
▪ Optimizing a position, an orientation and optionally a size of the femur implant, preferably and the size of the femur implant, relative to the native femur and patella based on the one or more morphological and/or biomechanical simulations;
▪ Optionally optimizing a position, an orientation and optionally a size of the tibia implant relative to the femur implant, femur implant position and patella, preferably the native femur, the native tibia and the native patella, based on the one or more morphological and / or biomechanical simulations; and
▪ Optionally, optimizing a position, an orientation and a size of the patella implant relative to the femur implant, the native femur, the tibia implant, the native tibia based on the one or more morphlogical and / or biomechanical simulations; and
▪ Outputting patient-specific planning information to one or more of an extemal computing device, visual display and a computer-readable storage device;
characterized in that
▪ the morphological parameters include local morphological parameters of the femur, the tibia, the patella, optionally the fibula and optionally proximal and distal morphological parameters, preferably whereby the proximal morphological parameters include the parameters of the proximal femur and pelvis and whereby the distal morphological parameters include the parameters of the distal tibia, the distal fibula and optionally the bones of the ankle and the foot;
▪ the implant-related parameters include the prosthetic design-related parameters and the prosthetic position and orientation-related parameters; and/or
▪ the patient-specific planning information considers the relative position of the patella to the femur and optionally the tibia in function of the range of motion of the knee, preferably, the patient-specific planning information considers the relative position of the patella to the femur and the tibia in function of the range of motion of the knee.

Another aspect of the present invention is a computer-implemented method of planning knee replacement surgery, the method comprising:
▪ Receiving input related to an anatomy of a patient, wherein the input comprises imaging data related to the femur, the tibia, the patella, the hip, pelvis, the fibula, the ankle, and the bones in the foot;
▪ Generating a three-dimensional (3D) model of the anatomy based on the input, wherein the 3D model comprises a patella model, a model of the femur, the tibia, the fibula, the pelvis, the hip, the ankle and the bones in the foot;
▪ Characterizing a morphology of the patella, the femur, the tibia, the fibula, the hip, the pelvis, the ankle and the bones in the foot based on the 3D model;
▪ Determining one or more implant-related or morphological parameters based on one or more of the 3D model and the morphology;
▪ Performing one or more morphological and biomechanical simulations based on the one or more implant-related or morphological parameters, wherein the simulations include one or more poses of the patella-femoral and tibio-femoral joint through a range of motion thereof and a range of motion of the hip and the ankle;
▪ Optimizing a position, an orientation and a size of the femur implant relative to the native femur and patella based on the one or more morphological and/or biomechanical simulations;
▪ Optimizing a position, an orientation and optionally a size of the tibia implant relative to the femur implant, femur implant position and patella based on the one or more morphological and biomechanical simulations; and
▪ Outputting patient-specific planning information to one or more of an extemal computing device, visual display and a computer-readable storage device;
characterized in that
▪ the morphological parameters include local morphological parameters of the femur, the tibia, the patella and proximal and distal morphological parameters;
▪ the implant-related parameters include the prosthetic design-related parameters and the prosthetic position and orientation-related parameters; and
▪ the patient-specific planning information considers the relative position of the patella to the femur and optionally the tibia in function of the range of motion of the knee.

Another aspect is a computer-implemented method of planning knee replacement surgery, the method comprising:
▪ Receiving input related to an anatomy of a patient, wherein the input comprises imaging data related to the femur, the tibia, the patella and optionally the hip, pelvis, the fibula, the ankle, and/or the bones in the foot;
▪ Generating a three-dimensional (3D) model of the anatomy based on the input, wherein the 3D model comprises a patella model, a model of the femur, the tibia and optionally of the fibula, the pelvis, the hip, the ankle and the bones in the foot;
▪ Characterizing a morphology of the patella, the femur, the tibia, and optionally the fibula, the hip, the pelvis, the ankle and the bones in the foot based on the 3D model;
▪ Determining one or more implant-related or morphological parameters based on one or more of the 3D model and the morphology;
▪ Performing one or more morphological and/or biomechanical simulations based on the one or more implant-related or morphological parameters, wherein the simulations include one or more poses of the patella-femoral and tibio-femoral joint through a range of motion thereof and optionally a range of motion of the hip and/or the ankle;
▪ Optimizing a position, an orientation and optionally a size of the femur implant relative to the native femur and patella based on the one or more morphological and/or biomechanical simulations;
▪ Optionally optimizing a position, an orientation and optionally a size of the tibia implant relative to the femur implant, femur implant position and patella based on the one or more morphological and / or biomechanical simulations; and
▪ Outputting patient-specific planning information to one or more of an extemal computing device, visual display and a computer-readable storage device;
characterized in that
▪ the morphological parameters include local morphological parameters of the femur, the tibia, the patella and optionally proximal and distal morphological parameters;
▪ the implant-related parameters include the prosthetic design-related parameters and the prosthetic position and orientation-related parameters; and/or
the patient-specific planning information considers the relative position of the patella to the femur and optionally the tibia in function of the range of motion of the knee.

In one embodiment, the characterization of the morphology for each of the aforementioned bone models is done using statistical shape models. These statistical models are developed from a large series, typically 200 or more, preferably 500 or more and even more preferably 2000 or more of scans taken from patients with similar conditions. These statistical shape models allow to identify the shape variations between various patients and thus characterize the likelihood of an individual patient's specific anatomy in relation to a large cohort of patients with similar conditions, such as but not limited to patients with osteoarthritis. Additionally, these statistical shape models can also used to identify the unique position of a set of characteristic points, often referred to as landmarks, on the bone models. This can for instance be achieved by identifying the position of these characteristic points on the mean shape that is constructed as part of the statistical shape model and subsequently morphing this mean shape into the specific, patient-specific bone model. While the mesh is thereby rigidly and / or elastically deformed, the position of said characteristic points is morphed into the patient-specific bone model and can thus be identified for the individual patient. Said characteristic points can be used to characterize a set of angles or distances, referred to as morphological parameters. As part of the morphological simulations / analysis, these morphological parameters can be evaluated for an individual patient and compared to a library or distribution of historic values obtained from a reference population. A reference population is thereby defined as a population with similar disease characteristics (i.e. osteoarthritis) and / or demographics (e.g. age, gender, ...) as the patient under consideration.

In another aspect, the morphological parameters can be split by their anatomical position relative to the knee joint. Preferably, reference is made to proximal parameters located proximal to the knee and distal parameters more distal to the knee. Even more preferably, the local parameters refer to the parameters of the knee joint.

In another aspect, the proximal and distal anatomical parameters include one or more of the femoral head center, femoral torsion, the acetabular version, the femoral anteversion, the quadriceps tendon alignment, the Q-angle, the tibial torsion, the tibial tuberosity to trochlear groove distance and/or orientation, preferably wherein the proximal and distal anatomical parameters include at least one, even more preferably at least two, and even more preferably all the aforementioned proximal anatomical parameters.

In another asect the local anatomical parameters include one or more of the (surgical) patellar tilt, the trochlear angles, the (surgical) anterior trochlear angle, the lateral trochlear inclination, the sulcus angle, the trochlear depth and width, the patella trochlear angle, the lateral trochlear rim height, the medial trochlear rim height preferably wherein the local anatomical parameters include at least one, even more preferably at least two, and even more preferably all the aforementioned local anatomical parameters and even more preferably always includes the patellar tilt or the surgical patellar tilt.

In another aspect, the local anatomical parameters include one or more of the patellar thickness, the patellar ridge height, the patellar length, the patellar convexity angle, the patellar medial slope, the patellar lateral slope, the patellar ridge height, the patellar medial length, the patellar lateral length, the patellar orbital distance preferably wherein the local anatomical parameters include at least one, even more preferably at least two, and even more preferably all the aforementioned local anatomical parameters and even more preferably always includes the patellar orbital distance, the patellar convexity and the patellar length.

In another aspect, the prosthetic design related parameters include one or more of the trochlear angles, the sulcus angle, the trochlear depth, the trochlear width, the medial trochlear rim height, the lateral trochlear rim height and the anterior trochlear angle, and preferably wherein the prosthetic design related parameters include at least one, even more preferably at least two, and even more preferably all the aforementioned prosthetic design related parameters.

In another aspect, the prosthetic position and orientation related parameters include one or more of the optimal sizing of the femur, the femur varus/valgus orientation in the coronal plane, the femur intemal/external rotation in the axial plane, preferably the femur flexion in the sagittal plane, the femur mediolateral translation in any plane, femoral flexion and the femur antero-posterior positioning in the sagittal plane, the femur anterior-posterior sizing in any plane, the femur proximodistal position in the coronal plane, the tibia medio-lateral position in any plane, the tibial antero-posterior and mediolateral position in the axial plane, the tibial internal/external rotation in the axial plane, the tibial slope in the sagittal plane, the tibia proximodistal position, preferably the varus/valgus orientation in the coronal palne, the optimal sizing of the tibia implant, preferably the implant baseplate, the tibial insert thickness and the type of tibial insert (cruciate retaining, cruciate stabilised, medial stabilised, medial pivot, posterior stabilised), preferably wherein the prosthetic position and rotation related parameters include at least one, even more preferably at least two, and even more preferably all the aforementioned prosthetic position and orientation related parameters.

In another aspect, the prosthetic position and orientation related parameters include one or more of the optimal sizing of the patella implant component, the mediolateral translation of the patella component, the anterior-posterior translation of the patella component, the superior-inferior translation of the patella component, the tilt of the patellar component in the axial plane, the rotation of the component in the coronal plane and the tilt in the sagittal plane, preferably wherein the prosthetic position and rotation related parameters include at least one, even more preferably at least two, and even more preferably all the aforementioned prosthetic position and orientation related parameters. The axial plane is thereby defined by the mediolateral and anteroposterior axis attached to the patella, the coronal plane is defined by the mediolateral and superior-inferior or ridge axis attached to the patella and the sagittal plane is defined by the superior-inferior or ridge axis and the anterior-posterior axis attached to the patella.

In another aspect, the patient-specific planning information comprises predictive values for one or more of
▪ The femoral angles and bone resection values,
▪ The tibial angles and bone resection values,
▪ Optionally patellar angles and bone resection values,
▪ The tibio-femoral balance,
▪ The patella-femoral balance,
▪ The size of the femur and tibia and optionally the patella implant components,
▪ Optionally the accurate position of the femur and the tibia and optionally the patella implant in the 3dD space,
▪ Risks for post-operative patella-femoral joint problems,
▪ Prosthetic positions susceptible of causing post-operative patella-femoral joint problems,
▪ Optimized prosthetic position with respect of the patella-femoral joint, the optimized prosthetic position and the optimized orientation of the femur implant relative to the patella through the range of motion of the tibiofemoral joint,
▪ Optimized prosthetic position with respect of the patella-femoral joint, the optimized prosthetic position and the optimized orientation of the tibia implant relative to the patella position through the range of motion of the tibiofemoral joint,
▪ Optimized prosthetic design with respect of the patella-femoral joint, choosing from a database of available designs from different implant manufacturers,
▪ The need for a patellar resurfacing and associated patella implant position and / or removal of pathological bone segments such as but not limited to osteophytes;
▪ Tibiofemoral vs patellofemoral morphological conflict, and
▪ In case of non-resolution of the tibiofemoral vs patellofemoral morphologic conflict with a standard implant, indicating the requirements for an adapted or custom implant.

In another aspect, the optimized orientation ensures a sufficient average distance or gap GM or GL between the articulating surfaces of the patella and the femur implant to avoid post-surgical pain.

In one embodiment, the average distance or gap GM or GL are calculated from a morphological analysis using static 3D models of the femur and patella bones obtained through a computed tomography (CT) scan or magnetic resonance imaging (MRI). In this embodiment, the medial and lateral gap GM resp. GL preferably exceed 3 millimeter, more preferably these gaps exceed 5 millimeter and even more preferably these gaps are symmetrical in the medial and lateral aspect of the patellofemoral joint.

In another embodiment, the average distance or gap GM or GL are calculated from biomechanical simulations assessing the dynamic performance of the patellofemoral and tibiofemoral joint. In this embodiment the gaps GM and GL shall be symmetrical and range between 3 and 5 mm relative to the pre-operative condition whereby the gaps are measured between the regions of interest on the bone or implant component and whereby the biomechanical simulation model uses information from the preoperative 3D bone models in combination with cartilage information that may be determined directly or indirectly from pre-operative scans. A direct determination could involve the use of MRI scans to assess the thickness of the cartilage, where an indirect determination could include fitting a statistical shape model of a cartilage model onto the bony anatomy of the distal femur, tibia and patella using morphological information of said bony structures.

In another aspect, the implant related and the morphological parameters comprise joint surfaces derivable from the 3D model, wherein the joint surfaces include one or more articulating surfaces or one or more non-articulating surfaces or a combination of articulating and non-articulating surfaces, and preferably wherein the implant related and the morphological parameters preferably comprise one or more articulating surfaces.

In one aspect, the tibiofemoral versus patellofemoral conflict is characterized by a set of rotational axes that are defined by fitting geometrical shapes through the aforementioned articulating surfaces. A first rotational axis can be obtained by fitting a first cylindrical shape through the articulating areas of the distal femur that constitute the tibiofemoral joint. A second rotational axis can be obtained by fitting a second cylindrical shape through the articulating areas of the distal femur that constitute the patellofemoral joint. The relative orientation and position of these axes in the axial, sagittal and coronal plane can then be compared to the equivalent axes obtained from a given implant design or size to select the optimal implant component or identify the need for a patient-specific implant component should the deviation between the morphological characteristics of the patient and the available implant designs be indicative for inferior post-operative outcomes.

In another aspect, the implant-related parameters and the morphological parameters further include one or more of:
▪ The sulcus angle, wherein the sulcus angle describes the angle between the medial and lateral facet of the trochlea and the lateral trochlear inclination;
▪ The lateral trochlear inclination, wherein the lateral trochlear inclination describes the angle between the posterior condylar line and the line through the lateral facet of the trochlea;
▪ The distal medial femoral articulating surface, the distal lateral femoral articulating surface, the posterior medial femoral articulating surface and / or the posterior lateral femoral articulating surface;
▪ The depth and mediolateral position of the sulcus of the trochlea.

In another aspect, the input comprises one or more of X-ray imaging data, MRI imaging data, CT imaging data, ultrasound, and lidar, wherein preferably, the input comprises a 3D imaging data, even more preferably 3D CT imaging data.

In another aspect, the input additionally comprises real time measurements of implant related parameters or morphological parameters or a combination of implant related parameters and morphological parameters prior to or during surgery, preferably kinematic information related to the hip, tibiofemoral and / or patellofemoral joint.

In another aspect, the implant optimization includes the alignment of the tibial baseplate with respect to the trochlear groove orientation and associated patellar positions through the range of motion of the knee and morphological parameters describing the tibial torsion and / or rotational alignment.

In another aspect, the input data further comprises reference data of the knee and optionally the hip, the pelvis, the foot and the ankle, and wherein the simulation of the implant position and orientation aims at the reestablishment of the non-pathological knee based on the reference data of the knee and optionally the hip, the pelvis, the foot and the ankle.

In another aspect, any of the sulcus of the trochlea is positioned more laterally, preferally by a distance of 2 mm to 5 mm and the height of the lateral trochlear rim is lowered, preferably by a distance of 2 mm to 5 mm, for a morphology displaying valgus deformity or any other deformity characterized by the femoral neck offset, femoral torsion, ankle morphology, preferably distal fibula and tibia morphology, patellar trochlear angle, patellar tilt, gap between the medial and lateral patellofemoral articulating surfaces and / or acetabular version; and/or the femoral and tibial knee center location.

Or the equivalent for a varus deformed knee in relation to any of the sulcus of the trochlea, the medial trochlear rim and optionally the lateral trochlear rim.

Another aspect of the present invention is a system, the system being configured to execute the computer-implemented method of planning knee replacement surgery according to the invention.

### DETAILED DESCRIPTION OF THE INVENTION

The invention is now explained in further detail.

### Definitions

The term "implant" is used to refer to a prosthetic device or structure manufactured to replace or enhance a biological structure, in particular the knee joint. The term "implant" can refer to a femoral implant attached to the femur, a tibial implant attached to the tibia and optionally constructed as a tibial baseplate in combination with an insert component and / or a patella implant attached to the patella.

The term "real-time" is used to refer to calculations or operations performed on-the-fly as events occur or input is received by the operable system. However, the use of the term "real-time" is not intended to preclude operations that cause some latency between input and response, so long as the latency is an unintended consequence induced by the performance characteristics of the machine.

The singular forms "a," "an," and "the" include plural references unless the context clearly dictates otherwise. Unless defined otherwise, all technical and scientific terms used herein have the same meanings as commonly understood by one of ordinary skill in the art. Nothing in this disclosure is to be construed as an admission that the embodiments described in this disclosure are not entitled to antedate such disclosure by virtue of prior invention.

As used in this document, the term "comprising" means "including, but not limited to."

The term "proximal" or "distal" can refer to anatomical aspects of the patient located closer to or further away from the knee joint [1.1]as typically used in scientific literature related to the human anatomy.

### A computer-implemented method of planning knee replacement surgery

The computer implemented method of planning knee replacement surgery applies to both total and partial knee replacement as well as uni-compartmental knee replacement. Knee replacement can refer to medial, lateral, patellofemoral, or a combination thereof.

### Input

The input relates to an anatomy of a patient and comprises imaging data related to the knee, the hip, the pelvis and optionally the ankle, preferably related to the knee the hip, the pelvis and the ankle. The input includes the patella.

In one embodiment, the input comprises reference data of the knee and optionally the hip, the pelvis and the ankle, preferably of the knee, the hip, the pelvis and the ankle. The simulation of the femur implant position and orientation aims at the reestablishment of the non-pathological knee based on the reference data of the knee and optionally the hip, the pelvis and the ankle, preferably of the knee and the hip, the pelvis and the ankle.

Reference data may include a library of anatomical, medical imaging, pre-operative (functional) assessments or outcomes data from patients that have previously been diagnosed with similar conditions such as for instance osteoarthritis or from patients that previously underwent similar interventions such as for instance total knee replacement surgery.

In one embodiment, the input comprises one or more of X-ray imaging data, MRI imaging data, and CT imaging data. In a preferred embodiment, the input comprises a 3D imaging data, even more preferably 3D CT imaging data.

In one embodiment, the input additionally comprises real time measurements of implant related parameters or morphological parameters or a combination of implant related parameters and morphological parameters during surgery, preferably kinematic information related to the hip, tibiofemoral and / or patellofemoral joint.

### 3D model

The three-dimensional (3D) model of the anatomy is based on the input. The 3D model comprises a patella model, a model of the femur, a model of the tibia and optionally a model of the pelvis, the hip, the fibula, the ankle and the bones from the foot.

### Morphology, implant related and morphological parameters

The 3D model characterizes a morphology of the patella, the femur, the tibia, and optionally the ankle, the fibula, the hip, the pelvis and the bones from the foot such as the calcaneum and the second metatarsal bone. Preferably, the 3D model characterizes also the fibula and the pelvis.

The 3D model is used to determine one or more implant-related or morphological parameters based on one or more of the 3D models and the morphology.

The morphological parameters include local morphological parameters of the knee, and optionally proximal and distal morphological parameters of the ankle, the foot, the hip and the pelvis of a patient.

In a preferred embodiment, the proximal anatomical parameters include one or more of the femoral head center, femoral torsion, the acetabular version, the femoral anteversion, the quadriceps tendon alignment, the Q-angle. In a particularly preferred embodiment, the proximal anatomical parameters include at least one, even more preferably at least two, and even more preferably all the aforementioned proximal anatomical parameters.

In a preferred embodiment, the distal anatomical parameters include one or more of the tibial torsion, the tibial tuberosity to trochlear groove distance projected in the mediolateral and proximo-distal direction, and optionally the foot progression angle. In a particularly preferred embodiment, the distal anatomical parameters include at least one, even more preferably at least two, and even more preferably all the aforementioned distal anatomical parameters.

In a preferred embodiment, the local anatomical parameters include one or more of the (surgical) patellar tilt, the trochlear angles, the (surgical) anterior trochlear angle, the lateral trochlear inclination, the sulcus angle, the trochlear depth and width, the patellar trochlear angle, the lateral trochlear rim height, the medial trochlear rim height. In a particularly preferred embodiment, the local anatomical parameters include at least one, even more preferably at least two, and even more preferably all the aforementioned local anatomical parameters.

The implant-related parameters include the prosthetic design-related parameters and the prosthetic position-related parameters.

In a preferred embodiment, the prosthetic design related parameters include one or more of the trochlear angles, the sulcus angle and position, the trochlear depth, the trochlear width, the medial trochlear rim height, the lateral trochlear rim height and the anterior trochlear angle. In a particularly preferred embodiment, the prosthetic design related parameters include at least one, even more preferably at least two, and even more preferably all the aforementioned prosthetic design related parameters.

In a preferred embodiment, the femoral prosthetic position and implant related parameters include one or more of the optimal sizing of the femur, the femur varus/valgus orientation in the coronal plane, the femur intemation/external rotation in the axial plane, mediolateral translation in any plane, femoral flexion and the antero-posterior positioning in the sagittal plane, the anterior-posterior sizing in any plane, the proximodistal position in the coronal plane. In a particularly preferred embodiment, the femoral prosthetic position and implant related parameters include at least one, even more preferably at least two, and even more preferably all the aforementioned prosthetic position and implant related parameters.

In a preferred embodiment, the tibial prosthetic position and implant related parameters include one or more of the tibia medio-lateral position in any plane, the tibial antero-posterior and mediolateral position in the axial plane, the tibial internal/external rotation in the axial plane, the tibial slope in the sagittal plane, the tibia proximodistal position, the optimal sizing of the tibia implant, the tibial insert thickness and the type of insert (cruciate retaining, cruciate stabilised, medial stabilised, medial pivot, posterior stabilised). In a particularly preferred embodiment, the tibial prosthetic position and implant related parameters include at least one, even more preferably at least two, and even more preferably all the aforementioned prosthetic position and implant related parameters.

In a preferred embodiment, the implant related and the morphological parameters comprise joint surfaces derivable from the 3D model. The joint surfaces include one or more articulating surfaces or one or more non-articulating surfaces or a combination of articulating and non-articulating surfaces.

In a particularly preferred embodiment, the implant related and the morphological parameters preferably comprise one or more articulating surfaces.

In one embodiment, the implant-related parameters and the morphological further include one or more of:
▪ The sulcus angle, wherein the sulcus angle describes the angle between the medial and lateral facet of the trochlea and the lateral trochlear inclination;
▪ The lateral trochlear inclination, wherein the lateral trochlear inclination describes the angle between the posterior condylar line and the line through the lateral facet of the trochlea;
▪ The distal medial femoral articulating surface, the distal lateral femoral articulating surface, the posterior medial femoral articulating surface and / or the posterior lateral femoral articulating surface;
▪ The depth and mediolateral position of the sulcus of the trochlea.

In one embodiment, the parameters further include the patellar tilt and the surgical patellar tilt.

### Biomechanical simulations

The biomechanical simulations are based on the one or more implant-related or morphological parameters. The simulations include one or more poses of the knee or knee implant through a range of motion thereof while also accounting for the hip and ankle position during the simulation. The simulations are essential to describe the relative position of various articulating segments relative to each other, as optical tracking can be challenging in a surgical setting. Indeed, this is particularly relevant for the patellofemoral joint, where attaching optical markers to the bone can be challenging as there is a significant (post-operative) fracture risk associated with this while trackers also take away movement freedom that is essential to complete surgery.

### Optimizing a position, orientation and size, and optionally a type of the femur implant

The simulations are used to optimize a position, an orientation and size of the femur implant relative to the patella and tibia based on the one or more morphological and/or biomechanical simulations.

In one embodiment, the optimized femur implant type will be selected from the implant library. The 'type' refers to the brand of the implant (e.g. Stryker), the implant family (e.g. Persona) and constraint (e.g. cruciate retaining, cruciate substituting, medial pivot, cruciate stabilising, ...).

In one embodiment, the optimized femur implant position includes adjusting or correcting one or more of the varus, the valgus, internal rotation, external rotation, mediolateral translation in any plane, the femoral flexion, anterior-posterior position, the proximodistal position, the sizing, and the thickness of the planned resection of femur bone and cartilage.

In a preferred embodiment, the optimized orientation ensures a sufficient gap between the articulating surfaces of the patella and the femur implant to avoid post-surgical pain.

In a further preferred embodiment, the optimized femur implant position includes one or more of adjusting or correcting the mediolateral trochlear sulcus position or the medial or lateral trochlear rim height.

### Optimizing a position, orientation and sizing, and optionally the type of the tibia implant

The simulations are used to optimize a position, orientation and size of the tibia implant relative to the patella and femur based on the one or more morphological and/or biomechanical simulations.

In one embodiment, the optimized femur implant type will be selected from the implant library. The 'type' refers to the brand of the implant (e.g. Stryker), the implant family (e.g. Persona) and constraint (e.g. cruciate retaining, cruciate substituting, medial pivot, cruciate stabilising, ...)
In one embodiment, the optimized tibia implant position includes adjusting or correcting one or more of the tibia medio-lateral position in any plane, the tibial antero-posterior and mediolateral position in the axial plane, the tibial internal/extemal rotation in the axial plane, the tibial slope in the sagittal plane, the tibia proximodistal position, the optimal sizing of the tibia, the tibial insert thickness and the type of insert (cruciate retaining, cruciate stabilised, medial stabilised, medial pivot, posterior stabilised).

In one preferred embodiment the trochlear groove of the femoral component is aligned with the tibial tuberosity and the antero-posterior axis and rotation of the tibial component.

### Outputting patient-specific planning information

Patient-specific planning information is output to one or more of an extemal computing device, visual display and a computer-readable storage device.

The patient-specific planning information considers the relative position of the patella to the trochlear groove in function of the flexion of the knee.

In a preferred embodiment, the patient-specific planning information comprises predictive values for one or more of:
▪ Tibiofemoral bone resection values,
▪ Patellofemoral bone resection values,
▪ The tibio-femoral balance,
▪ The patella-femoral balance,
▪ The size, and optionally the type of the femur and tibia and optionally the patella implant components,
▪ Risks for post-operative patella-femoral joint problems,
▪ Prosthetic positions susceptible of causing post-operative patella-femoral joint problems,
▪ Optimized prosthetic position with respect of the patella-femoral joint, the optimized prosthetic position and the optimized orientation of the femur implant relative to the patella through the range of motion of the tibiofemoral joint,
▪ Optimized prosthetic position with respect of the patella-femoral joint, the optimized prosthetic position and the optimized orientation of the tibia implant relative to the patella position through the range of motion of the tibiofemoral joint,
▪ Optimized prosthetic design with respect of the patella-femoral joint, choosing from a database of available designs from different implant manufacturers,
▪ The need for a patellar resurfacing and associated patella implant position and / or removal of pathological bone segments such as but not limited to osteophytes;
▪ Tibiofemoral vs patellofemoral morphological conflict, and
▪ In case of non-resolution of the tibiofemoral vs patellofemoral morphologic conflict with a standard implant, indicating the requirements for an adapted or custom implant.

### System

A further aspect of the present invention is a system, the system being configured to execute and display the computer-implemented method of planning knee replacement surgery of the invention.

### BRIEF DESCRIPTION OF THE DRAWINGS

Further features and advantages will become apparent from the following detailed description, based on appended drawings:

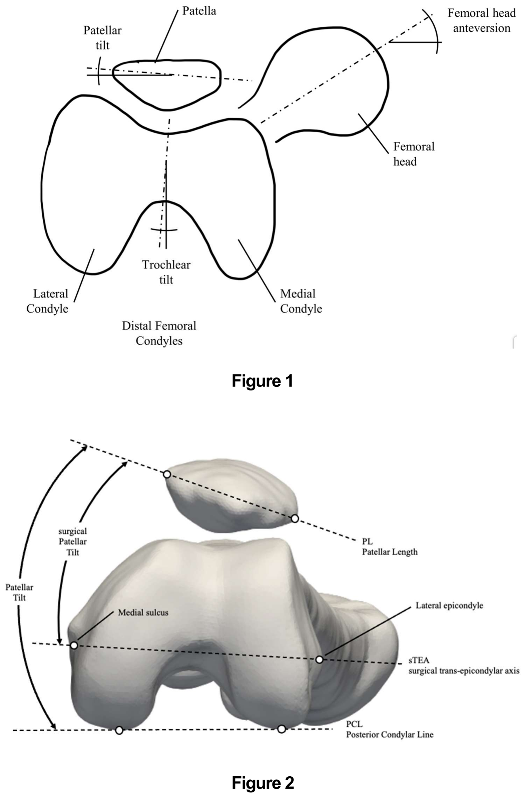

Figure 1 schematically illustrates the determination of characteristic points of the femur, the patella, and the femoral head, including the trochlear tilt, the patellar tilt and the femoral head anteversion. According to one embodiment of the present invention, these three parameters are balanced to ensure a sufficient gap between the patella and the femur implant through a range of motions of the knee. Figure 1 also applies to other knee anatomies.
Figure 2 schematically illustrates a model of a patella and the femur including three axes. A first axis characterizes the patellar length, a second axis characterizes the surgical trans-epicondylar axis, and a third axis characterizes the posterior condylar axis. The posterior condylar line is marked as PCL. PL describes the patellar length. The patellar length is determined by the mediolateral direction of the patella in the axial plane defined by the widest point on the medial and lateral reconstructed patella (after removal of the osteophytes). sTEA describes the surgical trans-epicondylar axis. The surgical trans-epicondylar axis describes the mediolateral direction of the distal femoral condyle defined by anatomical landmarks comprising the medial sulcus and the lateral epicondyle. PT describes the Patellar tilt. PCA describes the connection between the most posterior points on the medial resp. lateral femoral condyle in the axial plane. The patellar tilt is the angle between the posterior condylar line (PCL) and patellar line (PL). The surgical patellar tilt is the angle between the sTEA and the patellar length. Figure 2 also applies to other knee anatomies.

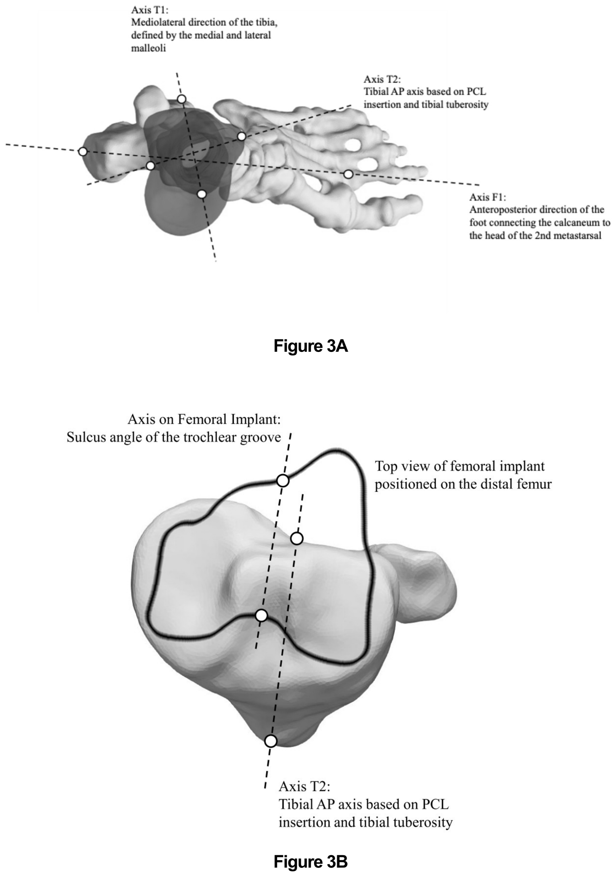

Figure 3A shows the mediolateral and anteroposterior axis of the distal anatomy defining the internal / external rotation and torsion of the distal knee, linked to the ankle and foot anatomy.
Figure 3B provides an illustration of the relationship between the anteroposterior axis of the proximal tibia and the orientation of the trochlear groove, optimized during the planning of the femoral component position.

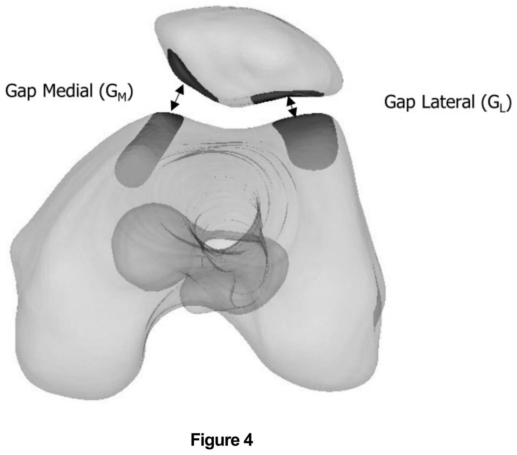

Figure 4 schematically illustrates the gap or distance between the patella and the femur implant. G_{M} describes the medial gap. G_{L} describes the lateral gap. Both the medial and the lateral gap depend on the position of the femur head, the hip, and the femur. The tight gap often causes short-term and/or long-term post-operative pain for the patient. Figure 4 also applies also to other knee anatomies.

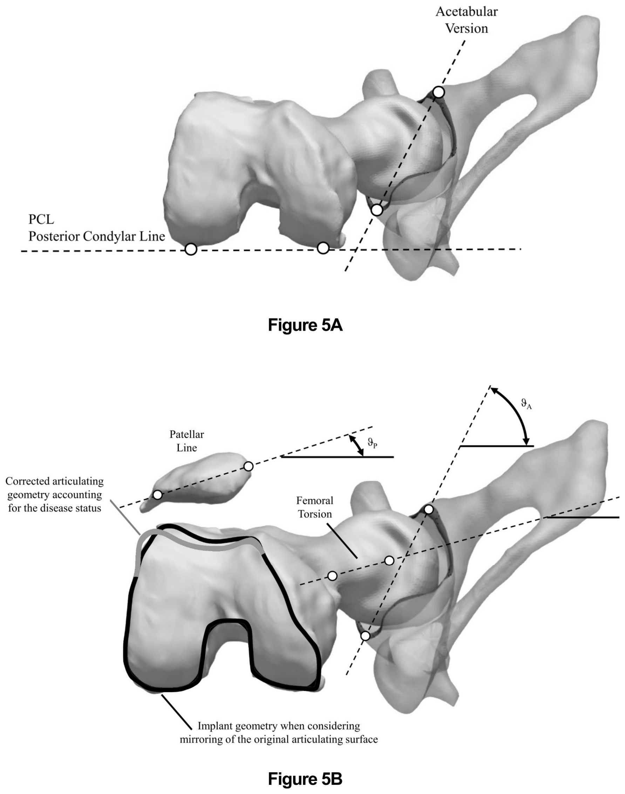

Figure 5A schematically illustrates the anatomical landmarks used to quantify the version of the hip joint.
Figure 5B schematically illustrates the hip anatomical parameters in conjunction with the distal femoral rotational parameters alongside a correction of the femoral implant and its effect on the trochlear groove relative to the intact condition directed by the torsion at the hip and / or the tilt of the patella.

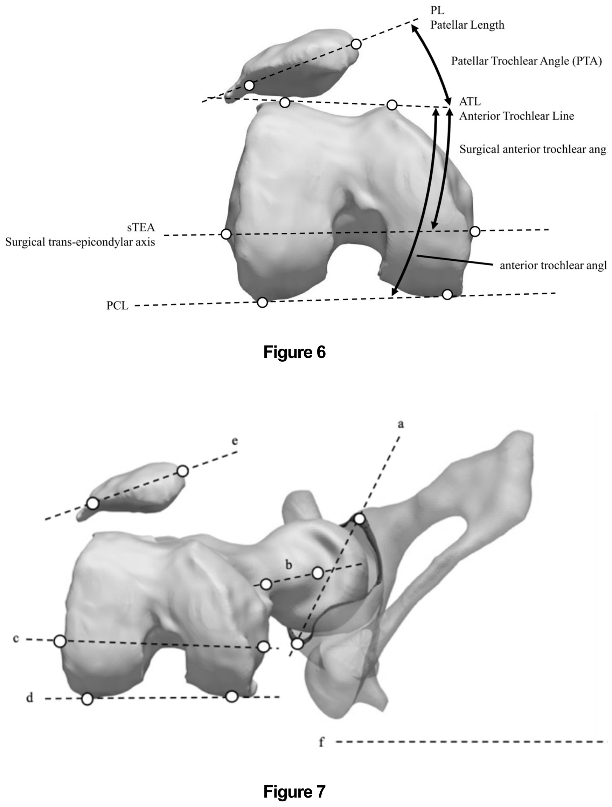

Figure 6 schematically illustrates the relative position of the patella to the femur. The line PL describes the patellar length as the mediolateral orientation of the patella measured at its broadest point. sTEA describes the surgical transepicondylar axis. ATL describes the anterior trochlear line, i.e. the line connecting the highest points of the medial and lateral trochlea in the axial plane at the level of the epicondyles. PCL describes the posterior condylar line. ATA describes the anterior trochlear angle, i.e. the angle between the anterior trochlear line and the posterior condylar line. sATA describes the surgical anterior trochlear angle, i.e. the angle between the anterior trochlear line and the surgical transepicondylar axis. PTA describes the patella trochlear angle, i.e. the angle between the patellar length and the anterior trochlear line.
Figure 7 schematically shows the combined hip, patella and distal femoral rotational alignment parameters used as input for the positioning of the femoral component.
Figure 8 shows a preferred embodiment of the invention according to which articulating and/or non-articulating surfaces are displayed. Target articulating zones are defined for the tibiofemoral joint and the patellofemoral joint. The sulcus designates the deepest point of the patella during articulation with counter-surface of the femoral implant. Visualization can happen through a 3D plot or a 2D plot with a heatmap or zones with similar radial distance from a reference axis comprising the surgical trans-epicondylar axis. The radial distance is derived from and defined by an accompanying 2D graph that describes the radial position perpendicular to said reference axis comprising the surgical trans-epicondylar axis of the bone or an equivalent axis on the implant at either the medial apex of the femoral condyle or the deepest point of the trochlear groove or a combination of both.

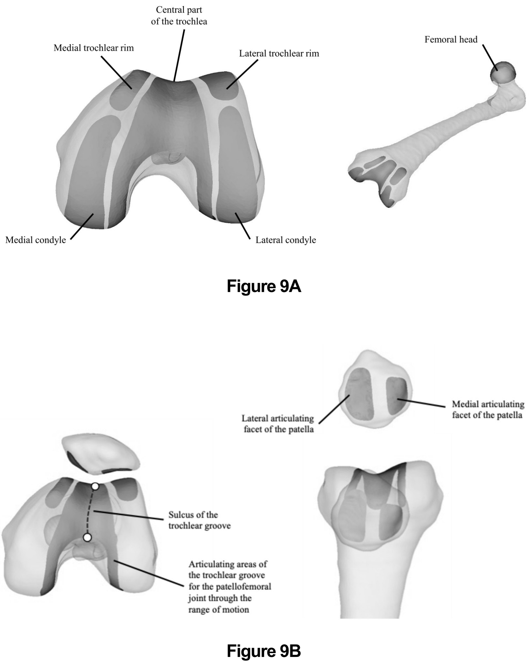

Figure 9A schematically illustrates the articulating surfaces of the femur, alternatively the femur implant and the femur head. The articulating surfaces are marked in dark grey. From upper left to right: 1, Medial trochlear rim, 2, central part trochlea 3, lateral trochlea rim. Lower left 4, medial condyle lower right: lateral condyle.
Figure 9B shows schematically the articulating surfaces of the patellofemoral joint with the identification of a medial and lateral articulating facet on the posterior side of the patella and the identification of an articulating area of the femur with the patella optionally identifying the sulcus point of the groove where the patella is optionally articulating beyond the anterior, trochlear groove on the femur and onto the condyles of the femur, as indicated by the extended area of the femur with a segment located on the central aspect of the medial and lateral condyles.

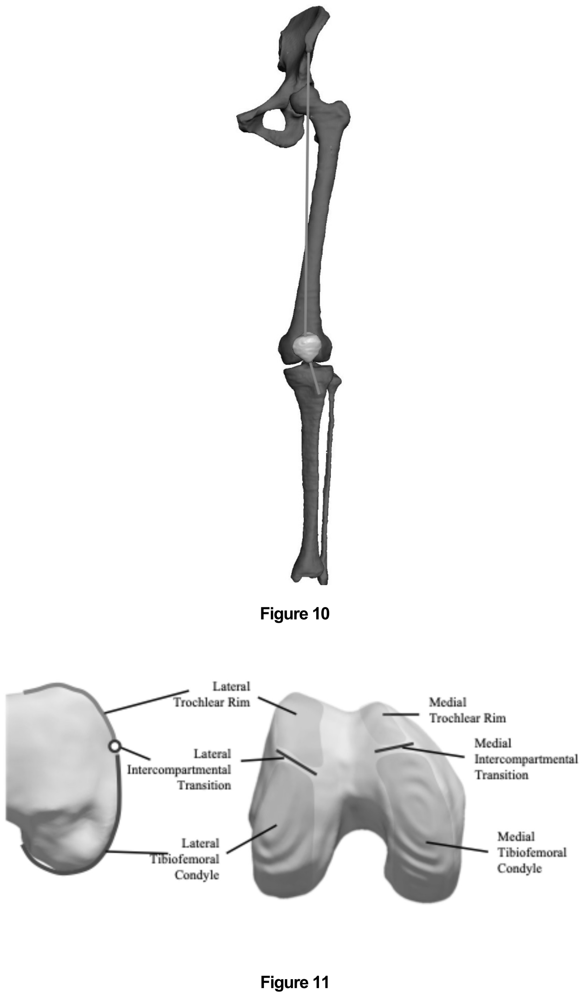

Figure 10 shows a schematic three-dimensional model of the lower limb including the distal tibia and fibula, the knee joint and the pelvis bone along with the proximal femur to allow for an anatomical description of the hip joint. Additionally, the figure shows the patella and a schematic representation of the key structures driving the position of the patella such as a first (infinitely stiff) connection between the patella and the tibial tubercle reflecting the patellar tendon and a second (elastically deforming) connection between the proximal patella and the pelvis reflecting the quadriceps muscle. These simplified representations are used as inputs for a patient-specific kinematic simulation model of the patellofemoral joint that helps define the articulating areas of the various joints and optionally the optimal placement and / or design of the implant components.
Figure 11 shows an embodiment of the method of the present invention including determining one or more of the lateral trochlear rim, the lateral intercompartmental transition, the lateral tibiofemoral condyle, medial trochlear rim, the medial intercompartmental transition, the medial tibiofemoral condyle. Figure 11 also applies to other knee anatomies.

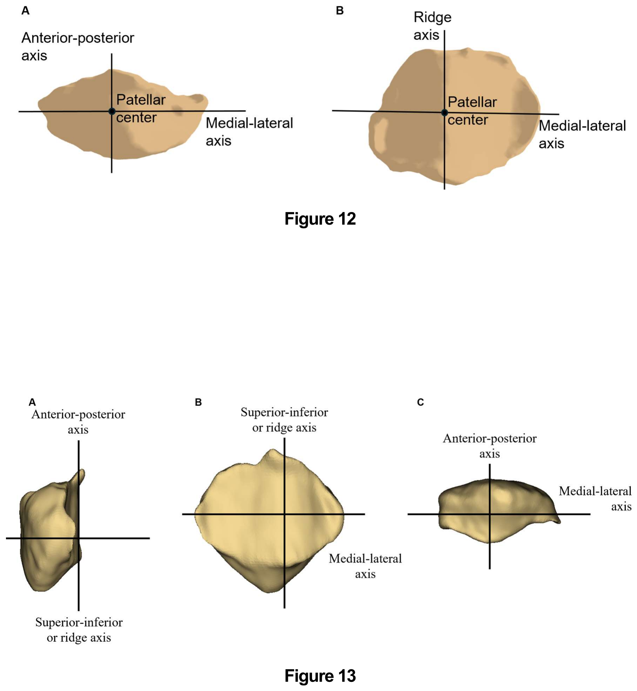

Figure 12A shows an embodiment of the method of the present invention including determining one or more of, preferably all of the patellar center, the anterior posterior axis and the medial-lateral axis (Axial plane view). Figure 12B shows an embodiment of the method of the present invention comprising considering one or more of, preferably all of the patellar center, the ridge axis and the medial-lateral axis of the patella (Coronal plane view).
   Accordingly, in one embodiment, the method of the present invention considers one or more of, preferably all of:
   ▪ the anterior-posterior axis is the third principal component of point cloud,
   ▪ the ridge axis is proximal distal, 1^{st} principal component of ridge landmark,
   ▪ the medial-lateral axis (ML) is a cross product of the ridge axis and the anterior-posterior axis,
   ▪ the PD axis is the cross product of the anterior-posterior axis and medial-lateral axis,
   ▪ the center point, the medial-lateral axis and the PD location are determined by using midpoint of ridge axis,
   ▪ Center point anterior-posterior axis (AP) location is the midpoint of the patellar anterior-posterior (AP) axis.
Figure 13 shows an embodiment of the method of the present invention including determining one or more of, preferably all of the anterior-posterior axis and the superior-inferior or ridge axis (Fig. 13A - sagittal plane view), the superior-inferior or ridge axis and the medial-lateral axis (Fig. 13B - coronal plane view), and the anterior-posterior axis and the medial-lateral axis (Fig. 13C - axial plane view). Figure 13 also applies to other knee anatomies.

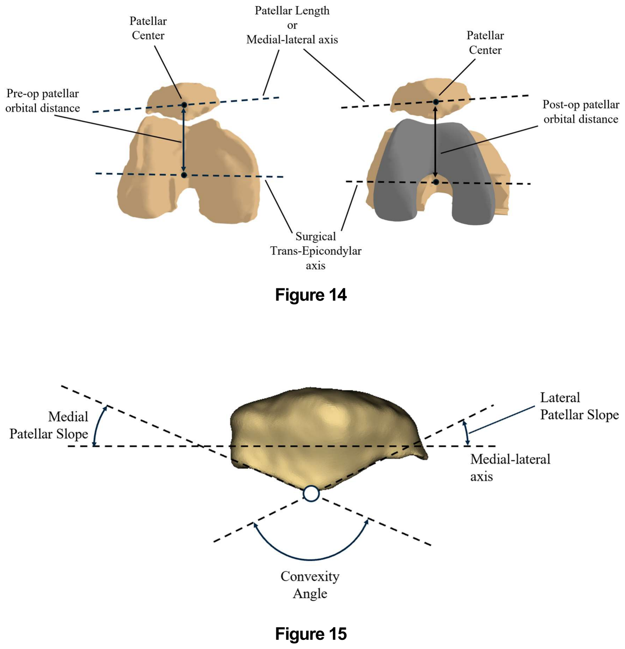

Figure 14 shows an embodiment of the method of the present invention including determining one or more of, preferably all of the pre-operative orbital distance, the patellar center, the patellar length, the medial-lateral axis, the surgical trans-epicondylar axis and the post-operative patellar orbital distance. Figure 14 also applies to other knee anatomies. Accordingly, Figure 14 presents an example of a 3D reconstruction of the lower limb anatomy obtained through a computed tomography scan with a focus on the knee joint but also considering the adjacent joints.
Figure 15 presents a coordinate system attached to the patella. This coordinate system looks at the patella in isolation and considers three mutually perpendicular axes along with an origin. Figure 15 also applies to other knee anatomies. Accordingly, in one embodiment, the method and the system of the invention comprises considering the medial patellar slope, the lateral patellar slope, the medial-lateral axis and the convexity angle.

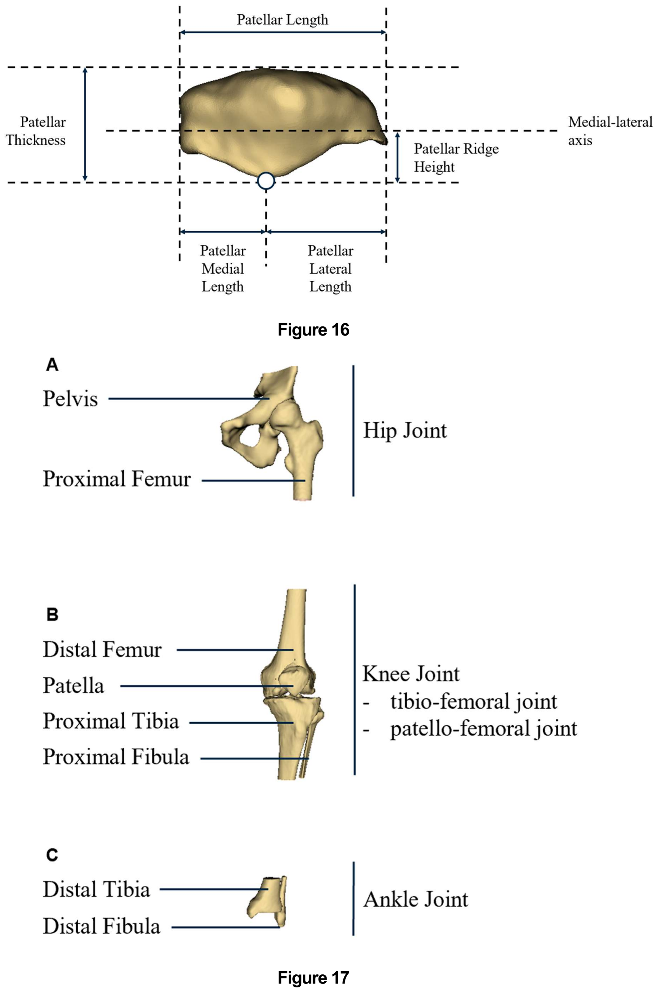

Figure 16 identifies and describes a series of local morphological parameters of the patella that can be used to characterize the morphology of the patella and optionally identify the optimal position, type and size of the patellar implant. Figure 16 also applies to other knee anatomies. Accordingly, in one embodiment, the method and the system of the invention comprises considering one or more, preferably all of the patellar length, the patellar thickness, the patellar ridge height, the medial-lateral axis, the patellar medial length and the patellar lateral length.
Figure 17 describes another specific patellofemoral morphological and / or biomechanical parameter, namely the patellar orbital distance which refers to the distance between the surgical trans-epicondylar axis on the femur and the patellar center. Figure 17 also applies to other knee anatomies. Accordingly, in one embodiment, the method and the system of the invention comprises considering one or more of, preferably all of
   ▪ The pelvis and the proximal femur (Fig. 17A - hip joint),
   ▪ The distal femur, patella, the proximal tibia, and the proximal fibula (Fig. 17B - knee joint, tibio-femoral joint, patella-femoral joint),
   ▪ The distal tibia and the distal fibula (Fig. 17C - ankle joint).

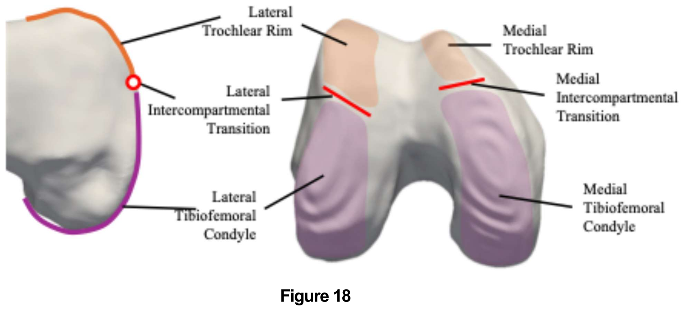

Figure 18 shows an embodiment of the method of the present invention including determining one or more of, preferably all of the lateral trochlear rim, the lateral intercompartmental transition, the lateral tibiofemoral condyle, the medial trochlear rim, the medial intercompartmental transition, and the medial tibiofemoral condyle. Figure 18 also applies to other knee anatomies.

### DETAILED DESCRIPTION OF THE DRAWINGS

### Figure 1

Figure 1 schematically illustrates the determination of characteristic points of the femur, the patella, and the femoral head, including the trochlear tilt, the patellar tilt and the femoral head anteversion. According to one embodiment the present invention, these three parameters are balanced to ensure a sufficient gap between the patella and the femur implant through a range of motions of the knee. Figure 1 also applies also to other knee anatomies.

### Figure 2

Figure 2 schematically illustrates the axial view of a 3D model of a patella and the femur including the axis 1 characterizing the patellar length (PL), axis 2 characterizing the distal femur mediolateral axis also referred to as the (surgical) trans-epicondylar axis (sTEA), and axis 3 characterizing the posterior femur mediolateral axis also referred to as the posterior condylar line (PCL).

In one embodiment, the relative positions of axis 1 (patellar length), axis 2 (distal femur mediolateral axis) and axis 3 (posterior femur mediolateral axis) are used to accurately assess the anatomy of the knee of the patient, more specifically, the anatomy of the patella and the trochlea and their relationship, such as neutral, tilted, or subluxed.

In one embodiment, the information of the relative positions of axis 1 (patellar length), axis 2 (distal femur mediolateral axis) and axis 3 (posterior femur mediolateral axis) are used to determine the alignment of the femoral component and asses the impact on the patella. The axes shown in Figure 2 are crucial for understanding the geometric relationships and angles that need to be maintained or corrected for optimal knee function during knee arthroplasty surgery. Specifically, the (surgical) patellar tilt angle (axis 1 relative to axis 2 or 3) helps the surgeon determine if there is any deviation that needs correction to ensure proper tracking of the patella within the trochlear groove (eg use extra extemal rotation of the femoral component). The mediolateral axes (axis 2 and 3) of the distal femur is essential for setting the correct rotational alignment of the femoral component of the knee implant with respect to the tibiofemoral joint. By using the 3D model and these axes, the surgeon can simulate various surgical scenarios, predict the impact of each scenario on the patella (and the rest of the knee for instance but not limited to the tibiofemoral joint), and customize the surgical approach to the patient's unique anatomy.

In one embodiment, the lines or axis (the terms "line" and "axis" are used synonymously) shown in Figure 2 are used to determine the exact position and orientation of the patella in relationship with the femur when planning a total knee arthroplasty (TKA) to ensure optimal alignment of the implant. In one embodiment, the axes shown in Figure 2 such as sTEA, and PCL are used to set the rotational alignment of the femoral component. A standard 3° of external rotations has typically been used for many years. Robotic systems now allow surgeons to tailor this to the individual anatomy of the patient. However, robotic system do not take the patella into account when planning. In one embodiment, the angles shown in Figure 2 such as the patellar tilt (PT) but also the angles that describe the trochlear orientation and depth of both native and prosthetic situation are used to plan for a more anatomic position and enhanced patellar tracking.

Note that the axis and situation in Figure 2 reflect a leg in full extension, however the above description also applies for various positions of the tibiofemoral joint through the range of motion such as for instance a range of knee flexion angles with the patella position moving relative to the distal femur as the knee flexes and extends.

### Figure 3A

Figure 3A schematically illustrates the measures used to characterize the distal aspects of the knee joint with a distal mediolateral axis defined by the medial and lateral malleoli and an anteroposterior axis at the level of the foot defined by the calcaneum and head of the second metatarsal bone. In relation to the anteroposterior axis of the knee, defined by the insertion of the posterior cruciate ligament and the tibial tubercle, the tibial torsion can be quantified. In one embodiment, the rotation at the proximal end of the tibia is described by the Akagi line, the line connecting the posterior apex of the medial and lateral tibial plateau or the connection between the geometric centers of the medial and lateral tibial plateau.

### Figure 3B

Figure 3B illustrates the link between this representation and the femoral implant placement. In one embodiment, the proximal tibial axis is compared to the anteroposterior axis of the femur, defined by the axis perpendicular to the axis connecting the centers of the medial and lateral tibiofemoral condyles but is alternatively defined by the trochlear angle through the range of motion. In one embodiment, this information is used to define the femoral implant rotational alignment in both the sagittal and axial plane, as this is linked to the varus / valgus and internal / extemal rotational alignment of the implant. This also applies also to other knee anatomies.

### Figure 4

Figure 4 schematically illustrates the gap between the patella and the femur implant. G_{M} describes the medial gap or distance. G_{L} describes the lateral gap distance. Both the medial and the lateral gap depend on the position of the femur head, the hip, the sacroiliac joint and the femur. The tight gap or distance often causes short-term and/or long-term post-operative pain for the patient. Figure 4 also applies also to other knee anatomies.

Accordingly, in one embodiment, the method of the present includes determining and/or optimizing the medial gap or distance G_{M} and the lateral gap or distance G_{L}. The medial and lateral gap are defined by evaluating the distance between the surface sections of interest, whereby the distance can be any of an extreme value, one or more parameters describing the distribution of distance or a distance along a normal direction from either of the articulating surfaces to the opposing surface.

### Figure 5

Figure 5A shows the acetabular version of the hip, defined by the tilt of the acetabular cup which in one embodiment is defined by fitting a plane through the rim of the acetabular cup and evaluating the direction of the normal to this rim in the axial plane of the pelvis. The horizontal, mediolateral direction is thereby defined by the distal femur such as for instance the posterior condylar line or altematively by landmarks of the pelvis such as for instance the normal to the pubic symphysis. In one embodiment, the information from the acetabular version is optionally used in combination with the torsion of the proximal femur as defined by the orientation of the femoral neck, the offset of the femoral neck and / or the tilt of the patella as illustrated in Figure 5B. The angle theta H hereby describes the proximal femoral version angle between the axis through the femoral neck and the posterior condylar line. The angle theta A describes the acetabular version defined by the orientation between the plane through the acetabular rim projected in the axial plane and the horizontal axis of either the pelvis or the knee defined for instance by the normal to the pubic symphysis. The angle theta P describes the patellar tilt, defined by the orientation between a first line connecting the medial and lateral apex of the patella and the posterior condylar line. The figure additionally shows the impact of these various angles on the femoral implant position and orientation. In one embodiment shown, the patellar tilt and / or combined version, optionally in relation to the overall limb alignment characterized for instance by the hip / knee / ankle angle, results in a lateral shift of the trochlear groove in combination with a lower lateral rim to allow for additional space for articulation of the patella. This is reflected by the grey circumferential line drawn on the distal femur, which clearly shows a shift relative to the black circumferential line. The latter represents the circumference of the intact, diseased femoral condyle. The former, grey, line shows the circumference of the corrected articulating surface that will be used as target for planning and optimizing the position of the femoral implant component. In one embodiment, a larger patellar tilt will result in a more pronounced mediolateral shift of the sulcus of the trochlea. A shift of 1 to 3 mm can be thought of in this regard.

### Figure 6

Figure 6 schematically illustrates the relative position of the patella to the femur. The patellar length PL is referred to as the mediolateral direction of the patella measured at its broadest point in the axial plane, optionally derived from a 3D reconstructed model. sTEA describes the surgical trans epicondylar axis, defined by the medial sulcus below the medial epicondyle and the lateral epicondyle. ATL describes the anterior trochlear line, i.e. the line connecting the highest points of the medial and lateral trochlea rim in the axial plane. PCL describes the posterior condylar line, connecting the most distal aspects of the medial and lateral condyle in the axial plane, alternatively connecting the most posterior aspects of the medial and lateral condyle in the axial plane. ATA describes the anterior trochlear angle or line, i.e. the angle between the anterior trochlear angle and the posterior condylar line. sATA describes the surgical anterior trochlear angle, i.e. the angle between the anterior trochlear line and the surgical trans-epicondylar axis. PTA describes the patella trochlea angle, i.e. the angle between the patellar length and the anterior trochlear line.

In one embodiment, the angles shown in Figure 6 are used to determine the relationship of the femur and the patella and the hip. Alternatively, these angles are used to define the femoral implant position, such as for instance shown in Figure 5B whereby the anterior trochlear line is rotated for the planned femur implant position to accommodate for the patellar tilt observed in the intact, diseased knee.

### Figure 7

Figure 7 schematically illustrates the relative position of the patella to the femur implant in function of the hip. The axis "a" describes the axis connecting the anterior and posterior margins of the acetabulum. The line "F" represents the transverse plane of the pelvis, i.e. a line a line, preferably a line, drawn through both the medial and lateral acetabular rims. The angle "AF" represents the acetabular version, i.e. the angle between the line connecting the anterior and posterior margins of the acetabulum (a) and the line representing the transverse plane of the pelvis (f), i.e. a line drawn through both acetabular rims or a line connecting both right and left anterior superior iliac spines (ASIS) or a line connecting both. The angle "BF" describes the femoral anteversion. The angle "BD" represents the femoral version. The axis "b" describes the femoral neck axis. The axis "c" describes the surgical trans-epicondylar axis. The axis "d" describes the posterior condylar axis of the distal femur. Figure 7 also applies to other knee anatomies. The angle "BC" corresponds to the surgical femoral version, i.e. the angle between b and c. The angle BE describes the angle between the femoral neck axis "b" and the patellar length "e".

In one embodiment, the angles and lines shown in Figure 7 are used to determine the relationship of the femur and the patella and the hip.

### Figure 8

Figure 8 shows a preferred embodiment of the invention including articulating and non-articulating surfaces. The visualization is derived from a 3D reconstructed model of the implant or altematively the distal femur joint, by considering radial slices that are extract around for instance the mediolateral surgical trans epicondylar axis. Target articulating zones are defined for the tibiofemoral joint and the patellofemoral joint. The sulcus designates the deepest point during articulation with counter-surface. Visualization can happen through a 3D plot or a 2D plot with a heatmap or zones with similar radial distance from a mediolateral axis such as but not limited to the surgical trans-epicondylar axis. Or altematively describing the distance between intact articulating surfaces and the implanted articulating surfaces. The radial distance is derived from and defined by an accompanying 2D graph that describes the radial position from the axis of the bone or implant at either the medical apex of the femoral condyle or the deepest point of the trochlear grove or a combination of both. An embodiment as shown in Figure 8 can be derived for a pathological knee and / or an implant. When both are available, a kinematic model of the implant can help identify the articulating zones of the implant with the tibial resp. patellar counter-surface and similarly a kinematic model of the intact, pathological knee can be used to detect the articulating zones of the intact knee. The articulating zones of the intact knee can be further corrected for the local disease status along or within these articulating zones either individually or collectively. Subsequently, an optimization of the implant position can be achieved by defining a loss function that accounts for the offset between both articulating areas in the radial direction as well as in the medio-lateral direction for a given implant and pathological condition. This loss function could favor either / or the tibiofemoral or patellofemoral joint articulating surface and optionally non-articulating surfaces to avoid bone overhang of the implant and associated soft-tissue impingement in these zones. Various implant geometries will have their unique representation. Accordingly, in one embodiment the aforementioned loss function can be used to select and / or suggest the implant size / type / modality with the optimized loss. In one embodiment, the optimized position for the implant is translated to resection depths or volume for the pathological bones

### Figure 9

Figure 9 shows the various articulating and non-articulating surface landmarks that are used to define the morphology of the knee and which are optionally also used as inputs for the simulation model that is used to define the relative position between both femur, patella and tibia. The areas of the distal femur are hereby subdivided in five distinct areas reflecting the medial trochlear rim, the central part of the trochlea and the lateral trochlear rim. These areas are used to describe the patellofemoral articulation and the relative position of the femur with respect to the patella. The center part of the trochlea is hereby not limited to the grooved section but optionally extends onto the central aspects of the medial and lateral femoral condyles. Using the surface of the center part of the trochlea, the sulcus line defining the deepest point can optionally be identified and marked as shown in Figure 9B. Additionally, the articulating areas of the tibiofemoral joint are identified reflected by the medial and lateral condyle as shown in Figure 9A. To characterize the hip joint, the articulating area on the femoral head is identified. Figure 9B additionally identifies the medial and lateral articulating facet of the patella. Using the surface landmarks described herein, the position of the femoral implant component can be identified and optimized for, whereby the offset in various articulating areas can be calculated. Optionally, an offset from these articulating surfaces can be planned for to accommodate for the diseased and deformed conditions, such as but not limited to lowering the lateral trochlear rim for patients that display a large patellar tilt.

### Figure 10

Figure 10 shows schematically a dynamic model of the knee where the patella is included in a simulation model to track the mediolateral and radial position of the patella in the trochlea groove. The attachment sites of the patella are connected on one hand to the tibial tuberosity and on the other hand to the acetabulum. The attachment of the quadriceps on the pelvis is identified by a location (relative to) the iliac spine and/or the acetabulum, and preferably the hip joint. Accordingly, in one embodiment, the method includes determining the attachment sites of the patella on the tibial tuberosity and on the acetabulum. The simulation model is further used to define the articulating surfaces of on the femur for either or both of the patellofemoral and tibiofemoral joint.

### Figure 11

Figure 11 identifies a number of articulating subareas at the distal femur whereby distinction is made between the tibiofemoral and patellofemoral articulating areas. These areas include the lateral trochlear rim, the lateral tibiofemoral condyle and their medial counterparts. The trochlear rim is separated from the tibiofemoral condyle by the intercompartmental transition line on the medial resp. lateral condyle. The identification of this transition line allows for a more straightforward identification of the medial and lateral trochlear rim and associated rim height. Both additionally allow to identify the trochlear groove more clearly, particularly in heavily wom or dysplastic joints. Splitting up the medial and lateral distal femoral condyles in a tibiofemoral and patellofemoral zone additionally allows to quantify the conflict between the tibiofemoral and the patellofemoral joint; identifying the delineation between the tibiofemoral and patellofemoral aspect allows to identify relevant areas on the intact femur in full extension and deep flexion. As such, when looking through the range of motion - for instance characterized by a rotation around the (surgical) epicondylar axis - the intersection with the relevant surface sections can be calculated for various knee flexion angles and the distance from the rotation axis to the surface sections can be evaluated to quantify the conflict between the tibiofemoral and patellofemoral joint, which would occur if such distances are deviating significantly from the design parameters of the implant.

### Figure 12

Figure 12A shows an embodiment of the method of the present invention including determining the Patellar center, the anterior posterior axis and the medial-lateral axis (Axial plane view). Figure 12B shows an embodiment of the method of the present invention comprising considering the patellar center, the ridge axis and the medial-lateral axis of the patella (Coronal plane view). Accordingly, in one embodiment, the method of the present invention considers one or more of:
▪ the anterior-posterior axis is the third principal component of point cloud,
▪ the ridge axis is proximal distal, 1^{st} principal component of ridge landmark,
▪ the medial-lateral axis (ML) is a cross product of the ridge axis and the anterior-posterior axis,
▪ the PD axis is the cross product of the anterior-posterior axis and medial-lateral axis,
▪ the center point, the medial-lateral axis and the PD location are determined by using midpoint of ridge axis,
▪ Center point anterior-posterior axis (AP) location is the midpoint of the patellar anterior-posterior (AP) axis.

### Figure 13

Figure 13 shows an embodiment of the method of the present invention including determining one or more of the anterior-posterior axis and the superior-inferior or ridge axis (Fig. 13A - sagittal plane view), the superior-inferior or ridge axis and the medial-lateral axis (Fig. 13B - coronal plane view), and the anterior-posterior axis and the medial-lateral axis (Fig. 13C - axial plane view).

### Figure 14

Figure 14 shows an embodiment of the method of the present invention including determining one or more of, preferably all of the pre-operative orbital distance, the patellar center, the patellar length, the medial-lateral axis, the surgical trans-epicondylar axis and the post-operative patellar orbital distance. Figure 14 also applies to other knee anatomies. Accordingly, Figure 12 presents an example 3D reconstruction of the lower limb anatomy obtained through a computed tomography scan with a focus on the knee joint but also considering the adjacent joints, namely the proximal hip joint and the distal ankle joint along with the relevant bone sections that constitute (part of) the joint.

### Figure 15

Figure 15 presents a coordinate system attached to the patella. This coordinate system looks at the patella in isolation and considers three mutually perpendicular axes along with an origin. Figure 15 also applies to other knee anatomies. Accordingly, in one embodiment, the method and the system of the invention comprises considering the medial patellar slope, the lateral patellar slope, the medial-lateral axis and the convexity angle. The anterior-posterior axis is the 3rd principal component of the point cloud that is obtained from uniformly (i.e. with an edge length in the range of 0.3 to 1.0 mm) remeshing the 3D reconstructed patella. The ridge in between the medial and lateral articulating aspect of the patella is identified as a landmark region constituting a series of points. The first principal component of this point cloud constitutes the ridge axis. The medio-lateral axis is the right-hand cross product between the ridge axis and the anterior-posterior axis. The proximal-distal axis is then obtained as the right-hand cross product between the anterior-posterior and medio-lateral axis. The center point is defined as the geometrical middle point on the ridge axis in the proximal-distal direction. In the anterior-posterior direction the center point is defined by either the middle point between the most anterior and most posterior point along the antero-posterior axis or altematively by the intersection between the most posterior position of the coronal plane that does not intersect the medial and lateral articulating surfaces.

### Figure 16

Figure 16 identifies and describes a series of local morphological parameters of the patella that can be used to characterize the morphology of the patella and optionally identify the optimal position, type and size of the patellar implant. The local morphological parameters consist of a series of angles Figure 15, derived from the geometry of the articulating surfaces in relation to a coordinate system such as for instance defined in the previous figure. The key angles include the slope in the axial plane of the medial and lateral articulating surfaces of the patellofemoral joint relative to the medio-lateral axis of the patella bone. Another key angle is the convexity angle describing the angle between the medial and lateral articulating areas in the axial plane of the patella. In addition, Figure 16 describes a series of geometrical length measures , such as the pateller length previously also described in Figure 2, the medial and lateral length of the patella and the ridge height and overall height of the patella.

### Figure 17

Figure 17 describes another specific patellofemoral morphological and / or biomechanical parameter, namely the patellar orbital distance which refers to the distance between the surgical trans-epicondylar axis on the femur and the patellar center. The surgical trans-epicondylar axis is defined in line with earlier figures such as Figure 2. The patellar center is defined in line with Figure 13 and located on the medio-lateral axis of the patella. In a first embodiment, the distance is calculated from reconstructed static images, such as a reconstructed computed tomography scan. More specifically, the distance is calculated in the axial plane of the patella. The latter can be varying dynamically as the knee (and the patella) moves through flexion-extension. When using dynamic imaging data, the orbital distance can be evaluated as function of the knee flexion angle. In a second embodiment, this orbital distance is also considered through the range of knee flexion and obtained from biomechanical simulations that are building on the patient-specific 3D models and simulating knee movement such as flexion / extension of the knee. Said orbital distance can thereby be compared between a pre-operative condition and a (simulated) post-operative condition to optimize the position, type and size of the femoral and / or patellar implant.

Figure 18 shows an embodiment of the method of the present invention including determining one or more of, preferably all of the lateral trochlear rim, the lateral intercompartmental transition, the lateral tibiofemoral condyle, the medial trochlear rim, the medial intercompartmental transition, and the medial tibiofemoral condyle. Figure 18 also applies to other knee anatomies.

## Claims

1. A computer-implemented method of planning knee replacement surgery, the method comprising:
▪ Receiving input related to an anatomy of a patient, wherein the input comprises imaging data related to the femur, the tibia, the patella, the hip, pelvis, the fibula, the ankle, and the bones in the foot;
▪ Generating a three-dimensional (3D) model of the anatomy based on the input, wherein the 3D model comprises a patella model, a model of the femur, the tibia, the fibula, the pelvis, the hip, the ankle and the bones in the foot;
▪ Characterizing a morphology of the patella, the femur, the tibia, the fibula, the hip, the pelvis, the ankle and the bones in the foot based on the 3D model;
▪ Determining one or more implant-related or morphological parameters based on one or more of the 3D model and the morphology;
▪ Performing one or more morphological and biomechanical simulations based on the one or more implant-related or morphological parameters, wherein the simulations include one or more poses of the patella-femoral and tibio-femoral joint through a range of motion thereof and a range of motion of the hip and the ankle;
▪ Optimizing a position, an orientation and a size of the femur implant relative to the native femur and patella based on the one or more morphological and/or biomechanical simulations;
▪ Optimizing a position, an orientation and optionally a size of the tibia implant relative to the femur implant, femur implant position and patella based on the one or more morphological and biomechanical simulations; and
▪ Outputting patient-specific planning information to one or more of an extemal computing device, visual display and a computer-readable storage device;
**characterized in that**
▪ the morphological parameters include local morphological parameters of the femur, the tibia, the patella and proximal and distal morphological parameters;
▪ the implant-related parameters include the prosthetic design-related parameters and the prosthetic position and orientation-related parameters; and
▪ the patient-specific planning information considers the relative position of the patella to the femur and optionally the tibia in function of the range of motion of the knee.

2. The computer-implemented method of any one of the preceding claims, wherein the proximal and distal anatomical parameters include one or more of the femoral head center, femoral torsion, the acetabular version, the femoral anteversion, the quadriceps tendon alignment, the Q-angle, the tibial torsion, the tibial tuberosity to trochlear groove distance and/or orientation, preferably wherein the proximal and distal anatomical parameters include at least one, even more preferably at least two, and even more preferably all the aforementioned proximal anatomical parameters.

3. The computer implemented method of any one of the preceding claims, wherein the local anatomical parameters include one or more of the (surgical) patellar tilt, the trochlear angles, the (surgical) anterior trochlear angle, the lateral trochlear inclination, the sulcus angle, the trochlear depth and width, the patella trochlear angle, the lateral trochlear rim height, the medial trochlear rim height preferably wherein the local anatomical parameters include at least one, even more preferably at least two, and even more preferably all the aforementioned local anatomical parameters and even more preferably always includes the patellar tilt or the surgical patellar tilt.

4. The computer-implemented method of any one of the preceding claims, wherein the prosthetic design related parameters include one or more of the trochlear angles, the sulcus angle, the trochlear depth, the trochlear width, the medial trochlear rim height, the lateral trochlear rim height and the anterior trochlear angle, and preferably wherein the prosthetic design related parameters include at least one, even more preferably at least two, and even more preferably all the aforementioned prosthetic design related parameters.

5. The computer-implemented method of any one of the preceding claims, wherein the prosthetic position and orientation related parameters include one or more of the optimal sizing of the femur, the femur varus/valgus orientation in the coronal plane, the femur internation/extemal rotation in the axial plane, the femur mediolateral translation in any plane, femoral flexion and the femur antero-posterior positioning in the sagittal plane, the femur anterior-posterior sizing in any plane, the femur proximodistal position in the coronal plane, the tibia medio-lateral position in any plane, the tibial antero-posterior and mediolateral position in the axial plane, the tibial internal/extemal rotation in the axial plane, the tibial slope in the sagittal plane, the tibia proximodistal position, the optimal sizing of the tibia implant, the tibial insert thickness and the type of tibial insert (cruciate retaining, cruciate stabilised, medial stabilised, medial pivot, posterior stabilised), preferably wherein the prosthetic position and rotation related parameters include at least one, even more preferably at least two, and even more preferably all the aforementioned prosthetic position and orientation related parameters.

6. The computer-implemented method of any one of the preceding claims, wherein the patient-specific planning information comprises predictive values for one or more of
▪ The femoral angles and bone resection values,
▪ The tibial angles and bone resection values,
▪ Optionally patellar angles and bone resection values,
▪ The tibio-femoral balance,
▪ The patella-femoral balance,
▪ The size of the femur and tibia and optionally the patella implant components,
▪ Optionally, the accurate position of the femur and the tibia and optionally the patella implant in the 3D space.
▪ Risks for post-operative patella-femoral joint problems,
▪ Prosthetic positions susceptible of causing post-operative patella-femoral joint problems,
▪ Optimized prosthetic position with respect of the patella-femoral joint, the optimized prosthetic position and the optimized orientation of the femur implant relative to the patella through the range of motion of the tibiofemoral joint,
▪ Optimized prosthetic position with respect of the patella-femoral joint, the optimized prosthetic position and the optimized orientation of the tibia implant relative to the patella position through the range of motion of the tibiofemoral joint,
▪ Optimized prosthetic design with respect of the patella-femoral joint, choosing from a database of available designs from different implant manufacturers,
▪ The need for a patellar resurfacing and associated patella implant position and / or removal of pathological bone segments such as but not limited to osteophytes;
▪ Tibiofemoral vs patellofemoral morphological conflict, and
▪ In case of non-resolution of the tibiofemoral vs patellofemoral morphologic conflict with a standard implant, indicating the requirements for an adapted or custom implant.

7. The computer-implemented method of any one of the preceding claims, wherein the optimized orientation ensures a sufficient average distance or gap G_{M} or G_{L} between the articulating surfaces of the patella and the femur implant to avoid post-surgical pain.

8. The computer-implemented method of any one of the preceding claims, wherein the implant related and the morphological parameters comprise joint surfaces derivable from the 3D model, wherein the joint surfaces include one or more articulating surfaces or one or more non-articulating surfaces or a combination of articulating and non-articulating surfaces, and preferably wherein the implant related and the morphological parameters preferably comprise one or more articulating surfaces.

9. The computer-implemented method of any one of the preceding claims, wherein the implant-related parameters and the morphological parameters further include one or more of:
▪ The sulcus angle, wherein the sulcus angle describes the angle between the medial and lateral facet of the trochlea and the lateral trochlear inclination;
▪ The lateral trochlear inclination, wherein the lateral trochlear inclination describes the angle between the posterior condylar line and the line through the lateral facet of the trochlea;
▪ The distal medial femoral articulating surface, the distal lateral femoral articulating surface, the posterior medial femoral articulating surface and / or the posterior lateral femoral articulating surface;
▪ The depth and mediolateral position of the sulcus of the trochlea.

10. The computer-implemented method of any one of the preceding claims, wherein the input comprises one or more of X-ray imaging data, MRI imaging data, CT imaging data, ultrasound, and lidar, wherein preferably, the input comprises a 3D imaging data, even more preferably 3D CT imaging data.

11. The computer-implemented method of any one of the preceding claims, wherein the input additionally comprises real time measurements of implant related parameters or morphological parameters or a combination of implant related parameters and morphological parameters prior to or during surgery, preferably kinematic information related to the hip, tibiofemoral and / or patellofemoral joint.

12. The computer-implemented method of any one of the preceding claims, whereby the implant optimization includes the alignment of the tibial baseplate with respect to the trochlear groove orientation and associated patellar positions through the range of motion of the knee and morphological parameters describing the tibial torsion and / or rotational alignment.

13. The computer-implemented method of any one of the preceding claims, wherein the input data further comprises reference data of the knee and optionally the hip, the pelvis, the foot and the ankle, and wherein the simulation of the implant position and orientation aims at the reestablishment of the non-pathological knee based on the reference data of the knee and optionally the hip, the pelvis, the foot and the ankle.

14. The computer-implemented method of any one of the preceding claims, wherein
▪ any of the sulcus of the trochlea is positioned more laterally and the height of the lateral trochlear rim is lowered for a morphology displaying valgus deformity or any other deformity **characterized by** the femoral neck offset, femoral torsion, ankle morphology, patellar trochlear angle, patellar tilt, gap between the medial and lateral patellofemoral articulating surfaces and / or acetabular version; and/or
▪ Or the equivalent for a varus deformed knee in relation to any of the sulcus of the trochlea, the medial trochlear rim and optionally the lateral trochlear rim.

15. A system, the system being configured to execute the computer-implemented method of planning knee replacement surgery according to any one of the preceding claims.
